# EUROPEAN PATENT SPECIFICATION

(11) **EP 3 960 212 B1**
(45) Date of publication and mention of the grant of the patent: **25.10.2023**
(21) Application number: 21188293.1
(22) Date of filing: 28.07.2021
(51) Int. Cl.: A61L 9/16, A61L 9/20, B01D 53/88, B01J 21/06, B01J 23/50, B01J 23/72, B01J 35/00, B01J 35/06

(54) **FILTERING DEVICE**
FILTRIERUNGSVORRICHTUNG
DISPOSITIF DE FILTRATION

(30) Priority: 28.08.2020 IT 202000004975 U
(43) Date of publication of application: 02.03.2022
(73) Proprietor: All Consulting S.r.l., 37060 Mozzecane (VR) (IT)
(72) Inventor: TAVELLA, Andrea, I-37060 Mozzecane (Verona) (IT)
(74) Representative: Frontoni, Stefano

(56) References cited:
- EP-A1- 0 937 398
- WO-A1-2010/016082
- WO-A2-2007/026387
- US-A1- 2009 123 343

## Description

The invention relates to a device for filtering fluids.

The device described herein may be used indifferently for filtering liquids and gases.

In general, in the art there exist devices of different types, which are associated to respective applications.

Such devices are distinguished from one another both from the standpoint of the operating principle and from the standpoint of the structure. WO 2007/026387 A2, WO 2010/016082 A1, US 2009/123343 A1 and EP 0 937 398 A1 are relevant prior art documents.

According to the application, the filtering device must feature a given efficiency and capacity of filtration. Moreover, its structural configuration must be defined also taking into account the constraints of encumbrance of the specific application.

In this context, the present invention proposes a new filtering device for elimination or abatement of volatile organic compounds and of pathogens within fluids.

In some embodiments, the filtering device described herein is extremely versatile, being able to adapt its own structural and operating characteristics according to the application at which it is aimed.

In particular, the invention described herein regards a filtering device according to Claim 1. The present invention moreover regards a filtering diaphragm according to Claim 8.

Further characteristics and advantages of the invention will emerge clearly from the ensuing description with reference to the annexed drawings, which are provided purely by way of non-limiting example and in which:
- Figure 1 is a perspective view of the device described herein according to one embodiment;
- Figure 2 is a cross-sectional view in the plane II-II of the device of Figure 1;
- Figure 3 is a perspective view of the device described herein according to a further embodiment;
- Figure 4 is a cross-sectional view in the plane IV-IV of the device of Figure 3;
- Figure 5 is a perspective view of the device described herein according to a further embodiment; and
- Figures 6 and 7 are two cross-sectional views in the planes VI-VI and VII-VII, respectively, of the device of Figure 5.

In the ensuing description, numerous specific details are provided to enable an in-depth understanding of the embodiments. The embodiments may be obtained without one or many of the specific details, or with other methods, components, materials, etc. In other cases, known structures, materials, or operations are not illustrated or described in detail so that the aspects of the embodiments will not be obscured.

Reference to "an embodiment" or "one embodiment" in the framework of the present description is intended to indicate that a particular configuration, structure, or characteristic described in relation to the embodiment is comprised in at least one embodiment. Hence, phrases such as "in an embodiment" or "in one embodiment" that may be present in various points of this description do not necessarily all refer to one and the same embodiment. In addition, the particular configurations, structures, or characteristics may be combined in any adequate way in one or more embodiments.

The references used herein are provided merely for convenience and do not define the sphere of protection or the scope of the embodiments.

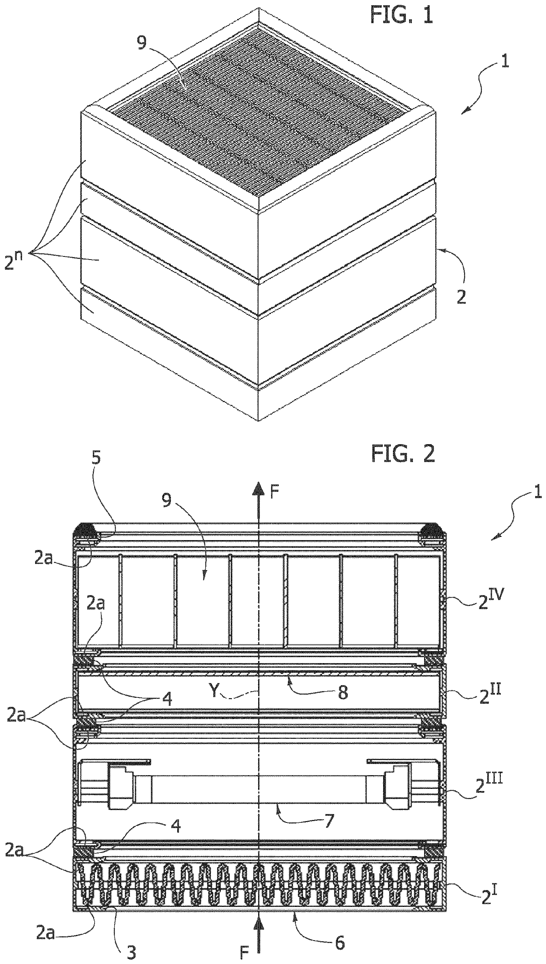

In Figures 1 and 2, designated by the reference 1 is a filtering device according to a first embodiment.

The above filtering device is preferably used for filtering air, in particular for eliminating or abating volatile and pathogens organic compounds present in the air.

The device represented comprises a casing 2 that has a generically tubular conformation about a reference axis Y and defines a passage of flow along the same axis Y for a flow of air F to be filtered.

In particular, as may be seen in Figure 2, the flow of air F enters the device through a bottom opening 3 and exits from a top opening 5.

In preferred embodiments, as, for example, in the one illustrated, the casing 2 is formed by a plurality of tubular sectors 2ⁿ that can be coupled together according to a modular construction.

In preferred embodiments, as, for example, in the one illustrated, the sectors 2ⁿ have a rectangular or square cross section and are made of aluminium.

Each sector 2ⁿ has opposite end edges constituted by tabs 2a bent back in a plane substantially orthogonal to the reference axis Y.

As may be seen in Figure 2, in the mounted condition of the device, the tubular sectors 2ⁿ are stacked on one another, resting on the adjacent sector or sectors with the edges or tabs 2a.

Gasket elements 4 are interposed between the tabs 2a of the adjacent sectors to guarantee hermetic seal of the walls defining the passage of the fluid through the casing 2.

Each sector 2ⁿ carries a functional element of the filtering device.

For this purpose, anchorage means are provided that fix the functional element to the inner walls of each sector. Such anchorage means may be of any known type and are consequently not described in detail herein.

The type of functional element and the combination of functional elements provided in the device may vary according to the applications.

In preferred embodiments, as, for example, in the one illustrated, a first sector 2^{I} has a filtering diaphragm 6 provided with an antipathogenic substance.

The antipathogenic substance comprises or is constituted by silver. Even more preferably, this compound is provided in the form of microparticles or nanoparticles.

Incidentally, it should be noted that by the term "antipathogenic substance" is here to be understood any substance capable of eliminating pathogens, such as viruses or bacteria, or at least inhibiting or eliminating the pathogenicity of these agents.

In preferred embodiments, the diaphragm 6 has a multilayer structure comprising a first layer that includes active carbons or zeolites, and a second layer that includes an antipathogenic substance and, preferably, also a photo-catalysing substance.

The photo-catalysing substance comprises or is constituted by zirconium oxynitride.

Preferably, the photocatalytic material is prearranged for constituting a matrix in which the nanoparticles of the antipathogenic material are englobed.

In this connection, provided hereinafter are some examples of compositions for producing the second layer in question provided with antipathogenic material and photo-catalytic material:
- zirconium oxynitride - silver (ZrNO-Ag);
- zirconium oxide (zirconia) - silver (ZrO₂-Ag) ;
- titanium dioxide - silver (TiO₂-Ag);
- titanium dioxide - zirconium oxide (zirconia) - silver (ZrO₂-TiO₂-Ag); and
- bismuth oxide - silver (Bi₂O₃-Ag).

Incidentally, it should be noted that bismuth oxide (Bi₂O₃), in addition to being photocatalytic, is markedly antipathogenic also by itself. The addition of silver makes it, however, possible to increase the antipathogenic properties of the overall layer.

In general, the second layer in question is able to perform an extremely effective action against organic substances and micro-organisms thanks to the combined action of the antipathogenic material and of the photo-catalytic material.

The filtering diaphragm 6 may present, as a whole, any shape, for example cylindrical, conical, parallelepipedal, spherical, etc. Preferably, the diaphragm is obtained in the form of pleated sheet material.

In preferred embodiments, as, for example, in the one illustrated, the device 1 comprises a second sector 2^{II} provided with a filtering diaphragm 8 of the same type as the filtering diaphragm 6.

In preferred embodiments, as, for example, in the one illustrated, the device 1 comprises a third sector 2^{III}, the functional element of which is constituted by at least one lamp 7 capable of emitting ultraviolet rays. Connection means of a known type are prearranged for connection of the lamp 7 to an electric-power source.

The third sector 2^{III} is arranged between the two sectors 2^{I} and 2^{II} in such a way that the lamp 7 is operatively associated to both of the filtering diaphragms 6 and 8 of the two sectors 2^{I} and 2^{II}. In particular, the lamp 7 is prearranged in a way that the ultraviolet rays emitted thereby strike at least a large part of the surfaces facing it of the filtering diaphragms 6 and 8.

Preferably, the diaphragms 6 and 8 are prearranged in such a way that the photo-catalysing substance faces the lamp 7.

According to mechanisms in themselves known, which will not be described in detail herein so as not to burden the present treatment, the ultraviolet rays activate the photo-catalysing substance, inducing an oxidative action by the substance itself in regard to the pollutant organic particles entrained by the air, which are to all effects destroyed by photocatalysis. The presence of the active carbon enables imprisonment in the diaphragm of such particles, thus guaranteeing completion of the oxidation processes.

In this connection, the layer of active carbons is preferably set upstream (with respect to the flow of air through the filtering diaphragm) the layer containing the photo-catalysing substance.

In addition, thanks to the processes of catalysis that destroy the imprisoned particles, the active carbons have the possibility of continuous self-regeneration.

The device 1 comprises a last sector 2^{IV} provided with a high-efficiency active-carbon filter 9. This filter basically has the function of absorbing the pollutant particles that have not been eliminated by the process of photocatalysis described above, as likewise of destroying the bacteria present in the air.

Elimination of the pathogenic microorganisms and of the organic particles, or in any case trapping thereof in the device, makes possible complete purification of the air and a considerable abatement of odours.

As emerges from the foregoing, in general the device described herein has a modular structure formed by a plurality of sectors, each with a respective functional element, which can be coupled together in the desired number and order, so that the device can assume the configuration best suited to the application for which it is designed.

For instance, in cases where high levels of efficiency of filtration are required, it is possible to provide a larger number of sectors than what is illustrated in Figures 1 and 2, to provide as a whole a larger number of filtering diaphragms, whether combined or not with a UV lamp.

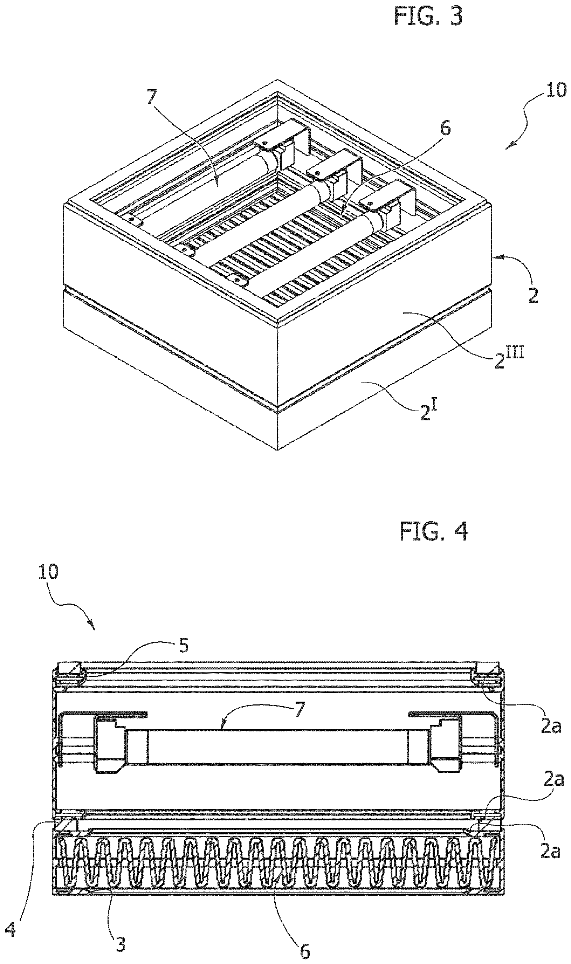

Figures 3 and 4 illustrate, instead, the device 1 described herein according to a more limited and compact configuration. In particular, according to this embodiment, the device 1 comprises just the two sectors 2^{I} and 2^{III} described above with reference to Figures 1 and 2. Preferably, the sector 2^{III} comprises a plurality of UV lamps 7 combined with the diaphragm 6. This configuration is, for example, particularly suitable for applications on air-conditioning systems of motor vehicles.

In some embodiments, also the structures of the device, such as the lamp-holder structure or the inner walls of the casing 2, may be coated with a photo-catalysing substance.

On the other hand, it is to be noted that the device described herein may be without ultraviolet lamp and function with solar light. Alternatively, a LED lamp or a lighting lamp of some other type can be provided instead of the ultraviolet lamp according to the type of photocatalytic material used.

To return to the filtering diaphragm, this can have a basic filtering layer, preferably made of nonwoven fabric (for example, spun-bonded, micro-spun-bonded, melt-blown nonwoven fabric, etc.) of synthetic material (for example polyethylene, polypropylene, polyester, polyamide, PTFE, etc.), glass microfibre, or else cellulose fibres.

Applied on the basic layer referred to are the layers described above, which are provided with active carbons, antipathogenic substance, and photo-catalysing substance.

For application of these layers one of the following methodologies for production of films of nanocomposites and/or microcomposites may be used:
∘ PVD (Physical Vapour Deposition) sputtering
∘ ultrasonic spray coating
∘ chemical spraying
∘ impregnation
∘ flocking
∘ silk-screen printing
∘ offset printing

The multilayer structure of the filtering diaphragm may vary according to the specific applications.

In particular, the various layers may be positioned with respect to one another in different arrangements and have variable thicknesses.

The thicknesses of the various photocatalytic and antipathogenic layers may range from a few nanometres (for example, 3 nm) up to a few hundreds microns (for example, 800 µm) according to the type of their formation and the method of deposition thereof.

Moreover, the basic filtering layer referred to may in turn comprise a plurality of layers constituted by different materials, or else further filtering layers may be provided positioned at various levels of the multilayer structure.

To return to the formation of the layers referred to above (provided with active carbons, antipathogenic substance, and photo-catalysing substance) on the basic filtering layer, some examples are given hereinafter.

### PVD sputtering of a photocatalytic and antipathogenic layer (nanoparticles)

As is in itself known, the PVD sputtering process envisages the use of a source (target) from which the material to be deposited on the substrate is made to vaporise via bombardment of particles carried out with plasma. In what follows the details of the process for froming layers of different compositions are provided.
1) zirconium oxynitride and silver (ZrNO-Ag)
   - zirconium (Zr) target
   - silver (Ag) target
   - gases (argon 90% - oxygen 5% - nitrogen 5%)
   - Magnetron power: ratio 100:1 (100 on zirconium target and 1 on silver target), and/or 200:1
2) zirconium oxide (zirconia) and silver (ZrO₂-Ag)
   - zirconium-oxide (ZrO₂) target
   - silver (Ag) target
   - gas (argon 100%)
   - radio-frequency power: ratio 100:1 (100 on zirconium-oxide (zirconia) target and 1 on silver
   target), and/or 200:1
3) bismuth oxide and silver (Bi₂O₃-Ag)
   - bismuth-oxide (Bi₂O₃) target
   - silver (Ag) target
   - gas (argon 100%)
   - radio-frequency power: ratio 100:1 (100 on bismuth-oxide target and 1 on silver target), and/or 200:1

### Deposition by means of flocking of photocatalytic and antipathogenic layer (microparticles)

The process envisages the use of bismuth-oxyhalogenide powders (BiOCl₁-xBrₓ, and/or Bi-doped BiOCl₁-xBrₓ) (grain size of the powder: from 1 um to 800 µm) and acrylic glue.

The process envisages the steps of:
- spreading the glue on the basic filtering layer made of nonwoven fabric by means of a process of silk-screen printing or offset printing;
- supplying the bismuth-oxyhalogenide powder by means of spray flocking or by means of deposition with mechanical systems (vibrating sieves, special rollers, etc.) so that it is spread evenly over the layer of glue previously applied;
- drying in an oven for formation of the photocatalytic and antipathogenic layer; and
- removing the excess powder using a suction fan or some other device.

### Deposition by means of silk-screen printing or offset printing of photocatalytic and antipathogenic layer (microparticles)

The process envisages the use of bismuth-oxyhalogenide powder (BiOCl₁-xBrₓ, and/or Bi-doped BiOCl₁-xBrₓ) (grain size of the powder: from 1 um to 800 µm) and acrylic glue.

The process envisages the following steps:
- appropriately mixing the bismuth-oxyhalogenide powder and the acrylic glue to create a fluid paste;
- spreading the paste over the basic filtering layer made of nonwoven fabric by means of a process of silk-screen printing or offset printing; and
- drying in an oven.

The processes referred to above are provided merely by way of example.

As already mentioned above, the device described herein may also be used for filtering liquids.

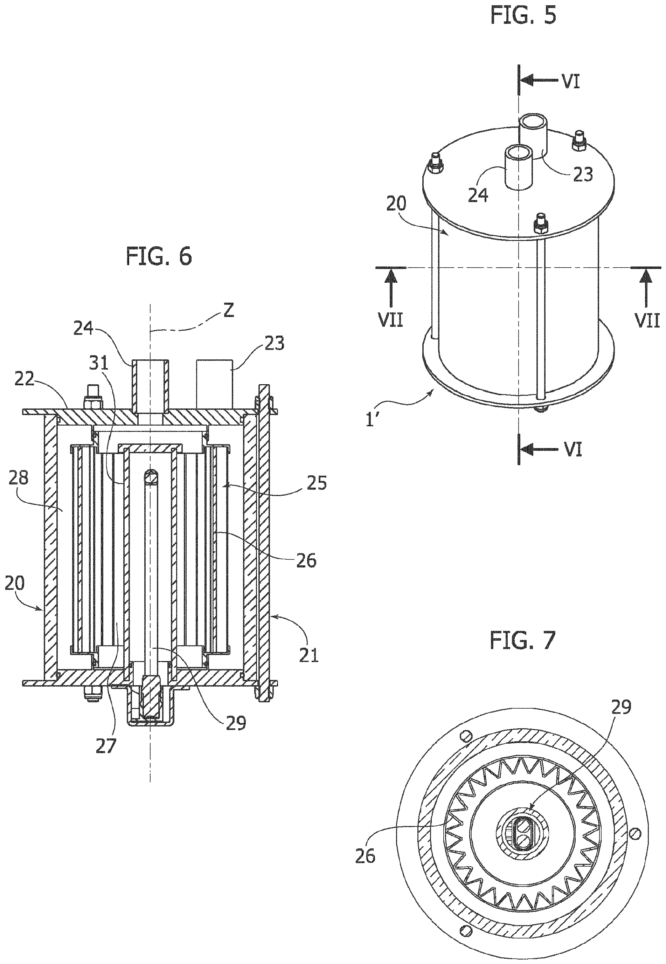

In this connection, Figures 5, 6, and 7 illustrate the device according to an embodiment for such an application.

According to this variant embodiment, the filtering device, designated as a whole by the reference number 1', comprises a casing 20 formed by a substantially cylindrical container body 21 and a lid 22. Defined on the lid 22 are an inlet 23 and an outlet 24 for the liquid to be filtered.

Set within the casing 20 is a filter cartridge 25, which has a tubular shape and is positioned about the reference axis Z of the container body 21.

The cartridge 25 comprises a filtering diaphragm 26 of the same type as the filtering diaphragms 6 and 8 described previously with reference to Figures 1 and 2.

The cartridge 25 defines within the casing 20 an inner chamber 27, which is separated from a region 28 external to the cartridge 25 by the filtering diaphragm 26.

Preferably, the cartridge 25 is carried by the lid 22, which is connected via fast-coupling connection means.

The inlet 23 is positioned on the lid in such a way that the liquid that flows in the casing 20 is located within the external region 28, whereas the outlet 24 is positioned so as to receive the liquid contained in the inner chamber 27.

Consequently, during operation, the liquid enters through the inlet 23, traverses the filtering diaphragm 26 entering the chamber 27, and finally exits through the outlet 24.

The device 1 further comprises an ultraviolet lamp 29, which is inserted within the chamber 27 and is surrounded by a sleeve 31 prearranged for closing it hermetically with respect to the liquid that, during operation, traverses the chamber itself.

Preferably, the lamp 29 is carried by the bottom of the container body 21, also provided on which are means for connection of the lamp itself to an electric-power source.

The lamp 29 is operatively associated to the filtering diaphragm 26 so that it can work according to the operation previously described with reference to Figures 1 and 2.

In view of the foregoing, it is now clear that the present invention can be obtained according to multiple configurations, thus making it possible to satisfy the requirements of different sectors of application.

In each configuration, the device described herein comprises a casing defining a passage of flow, which, during operation of the device, is to be traversed by a fluid to be filtered, and a filtering diaphragm mounted within the casing to carry out an action of filtering of the fluid that traverses the passage of flow, the filtering diaphragm being provided with an antipathogenic substance.

Preferably, the filtering diaphragm is also provided with a photo-catalysing substance, and the device may comprise an ultraviolet lamp operatively associated to the filtering diaphragm.

The present applicant has found that the filtering device 1, in particular in the embodiment in which the filtering diaphragm is provided both with an antipathogenic substance and with a photo-catalysing substance and is associated to an ultraviolet lamp, is extremely versatile and is able to satisfy the requirements of an extremely wide range of applications.

For instance, the device described herein may be used in any of the following sectors: automotive, transport, agricultural machinery, air-conditioning systems and/or HVAC systems, systems for treating technical gases, drinking water, industrial water, beverages in general, etc.

## Claims

1. A device (1; 1') for filtering a fluid, comprising a casing (2; 20) defining a passage of flow, which, during operation of the device, is to be traversed by a fluid (F) to be filtered, and a filtering diaphragm (6, 8; 26) mounted within the casing (2; 20) to carry out an action of filtering of the fluid that traverses the passage of flow, the device being **characterised in that** the filtering diaphragm (6, 8; 26) is provided with an antipathogenic substance,
wherein the filtering diaphragm comprises a basic layer, which includes one or more of the following materials: nonwoven fabric made of synthetic material, glass microfibre, impregnated cellulose fibres,
wherein, on the basic layer of the filtering diaphragm, zirconium oxynitride as photo-catalysing substance, and silver as antipathogenic substance are applied via PVD sputtering,
wherein the photo-catalysing substance constitutes a matrix englobed in which are particles of the antipathogenic substance.

2. The device according to Claim 1, the device further comprising at least one lamp (7; 29) designed to emit ultraviolet rays, which is operatively associated to said filtering diaphragm (6, 8; 26).

3. The device according to Claim 1 or Claim 2, wherein the filtering diaphragm (6, 8; 26) has a multilayer structure comprising a first layer provided with active carbons and a second layer provided with the antipathogenic substance and, preferably, also with the photo-catalysing substance.

4. The device according to any one of the preceding claims, further comprising an active-carbon filter mounted within the casing to carry out an action of filtering on the fluid that traverses the passage of flow.

5. The device according to any one of the preceding claims, wherein said casing (2) comprises a modular structure including a plurality of tubular sectors (2^{I,} 2^{II}, 2^{III}, 2^{IV}), which can be coupled together at end edges, on each sector there being mounted a functional element selected from among said ultraviolet lamp (7; 29), said filtering diaphragm (6, 8; 26), and said active-carbon filter (9).

6. The device according to Claim 5, wherein said sectors (2^{I,} 2^{II}, 2^{III}, 2^{IV}) can be coupled together in series along a reference axis (Y), in the desired number and order.

7. The device according to either Claim 5 or Claim 6, wherein each sector comprises end edges constituted by tabs bent back (2a) in a plane substantially orthogonal to the reference axis (Y), a gasket element (4) being set between the end edges of two contiguous sectors.

8. A filtering diaphragm (6, 8; 26) for a device for filtering a fluid, comprising a basic layer, which includes one or more of the following materials: nonwoven fabric made of synthetic material, glass microfibre, impregnated cellulose fibres, and
wherein, on the basic layer of the filtering diaphragm, zirconium oxynitride as a photo-catalysing substance, and silver as an antipathogenic substance are applied via PVD sputtering, and
wherein the photo-catalysing substance constitutes a matrix englobed in which are particles of the antipathogenic substance.

9. The filtering diaphragm (6, 8; 26) according to Claim 8, having a multilayer structure that comprises a first layer provided with active carbons or zeolites, and a second layer provided with the antipathogenic substance and with the photo-catalysing substance.

## Patentansprüche

1. Vorrichtung (1; 1') zum Filtrieren eines Fluids, umfassend ein Gehäuse (2; 20), das einen Flussdurchgang definiert, der während des Betriebs der Vorrichtung von einem zu filtrierenden Fluid (F) durchquert werden muss, und ein Filterdiaphragma (6, 8; 26), das innerhalb des Gehäuses (2; 20) angebracht ist, um einen Vorgang des Filtrierens des Fluids, das den Flussdurchgang durchquert, durchzuführen, wobei die Vorrichtung **dadurch gekennzeichnet ist, dass** das Filterdiaphragma (6, 8; 26) mit einem antipathogenen Stoff versehen ist,
wobei das Filterdiaphragma eine Basisschicht umfasst, die eines oder mehrere der folgenden Materialien enthält: Vliesgewebe aus synthetischem Material, Glas-Mikrofasern, imprägnierten Cellulosefasern,
wobei auf der Basisschicht des Filterdiaphragmas Zirkoniumoxynitrid als photokatalysierender Stoff und Silber als antipathogener Stoff durch PVD-Sputtern aufgebracht sind,
wobei der photokatalysierende Stoff eine Matrix bildet, in der Partikel des antipathogenen Stoffs eingebettet sind.

2. Vorrichtung gemäß Anspruch 1, wobei die Vorrichtung ferner wenigstens eine Lampe (7; 29), die dafür gestaltet ist, Ultraviolettstrahlen zu emittieren und die funktionsfähig mit dem Filterdiaphragma (6, 8; 26) verbunden ist, umfasst.

3. Vorrichtung gemäß Anspruch 1 oder Anspruch 2, wobei das Filterdiaphragma (6, 8; 26) eine mehrschichtige Struktur aufweist, die eine erste Schicht, die mit Aktivkohle versehen ist, und eine zweite Schicht, die mit dem antipathogenen Stoff und, vorzugsweise, auch mit dem photokatalysierenden Stoff versehen ist, umfasst.

4. Vorrichtung gemäß einem der vorstehenden Ansprüche, ferner umfassend einen Aktivkohlefilter, der innerhalb des Gehäuses angebracht ist, um einen Vorgang des Filtrierens des Fluids, das den Flussdurchgang durchquert, durchzuführen.

5. Vorrichtung gemäß einem der vorstehenden Ansprüche, wobei das Gehäuse (2) eine modulare Struktur umfasst, die eine Mehrzahl von rohrförmigen Sektoren (2^{I}, 2^{II}, 2^{III}, 2^{IV}) enthält, die an Endkanten zusammengekoppelt werden können, wobei an jedem Sektor ein Funktionselement ausgewählt aus der Ultraviolettlampe (7; 29), dem Filterdiaphragma (6, 8; 26) und dem Aktivkohlefilter (9) angebracht ist.

6. Vorrichtung gemäß Anspruch 5, wobei die Sektoren (2^{I}, 2^{II}, 2^{III}, 2^{IV}) in der gewünschten Anzahl und Reihenfolge in Reihe entlang einer Referenzachse (Y) zusammengekoppelt werden können.

7. Vorrichtung gemäß einem von Anspruch 5 oder Anspruch 6, wobei jeder Sektor Endkanten umfasst, die von Laschen gebildet werden, die in einer Ebene im Wesentlichen senkrecht zu der Referenzachse (Y) zurückgebogen sind (2a), wobei zwischen den Endkanten von zwei benachbarten Sektoren ein Dichtungselement (4) angeordnet ist.

8. Filterdiaphragma (6, 8; 26) für eine Vorrichtung zum Filtrieren eines Fluids, umfassend ein eine Basisschicht, die eines oder mehrere der folgenden Materialien enthält: Vliesgewebe aus synthetischem Material, Glas-Mikrofasern, imprägnierten Cellulosefasern, und
wobei auf der Basisschicht des Filterdiaphragmas Zirkoniumoxynitrid als photokatalysierender Stoff und Silber als antipathogener Stoff durch PVD-Sputtern aufgebracht sind, und
wobei der photokatalysierende Stoff eine Matrix bildet, in der Partikel des antipathogenen Stoffs eingebettet sind.

9. Filterdiaphragma (6, 8; 26) gemäß Anspruch 8 mit einer mehrschichtigen Struktur, die eine erste Schicht, die mit Aktivkohle oder Zeolithen versehen ist, und eine zweite Schicht, die mit dem antipathogenen Stoff und mit dem photokatalysierenden Stoff versehen ist, umfasst.

## Revendications

1. Dispositif (1 ; 1') pour filtrer un fluide, comprenant un boîtier (2 ; 20) définissant un passage d'écoulement, qui, durant le fonctionnement du dispositif, doit être traversé par un fluide (F) destiné à être filtré, et un diaphragme de filtration (6, 8 ; 26) monté à l'intérieur du boîtier (2 ; 20) pour réaliser une action de filtration du fluide qui traverse le passage d'écoulement, le dispositif étant **caractérisé en ce que** le diaphragme de filtration (6, 8 ; 26) est pourvu d'une substance anti-pathogène,
dans lequel le diaphragme de filtration comprend une couche de base, qui inclut un ou plusieurs des matériaux suivants : un tissu non tissé fait de matériau synthétique, une microfibre de verre, des fibres de cellulose imprégnées,
dans lequel, sur la couche de base du diaphragme de filtration, de l'oxynitrure de zirconium, en tant que substance photo-catalytique, et de l'argent, en tant que substance anti-pathogène, sont appliqués par l'intermédiaire de pulvérisation PVD,
dans lequel la substance photo-catalytique constitue une matrice englobée dans laquelle sont des particules de la substance anti-pathogène.

2. Dispositif selon la revendication 1, le dispositif comprenant en outre au moins une lampe (7 ; 29) conçue pour émettre des rayons ultraviolets, qui est fonctionnellement associée audit diaphragme de filtration (6, 8 ; 26).

3. Dispositif selon la revendication 1 ou la revendication 2, dans lequel le diaphragme de filtration (6, 8 ; 26) a une structure multicouche comprenant une première couche pourvue de charbons actifs et une seconde couche pourvue de la substance anti-pathogène et, de préférence, également de la substance photo-catalytique.

4. Dispositif selon l'une quelconque des revendications précédentes, comprenant en outre un filtre à charbon actif monté à l'intérieur du boîtier pour réaliser une action de filtration sur le fluide qui traverse le passage d'écoulement.

5. Dispositif selon l'une quelconque des revendications précédentes, dans lequel ledit boîtier (2) comprend une structure modulaire incluant une pluralité de secteurs tubulaires (2^{I}, 2^{II}, 2^{III}, 2^{IV}), qui peuvent être accouplés les uns aux autres au niveau de bords d'extrémité, sur chaque secteur étant monté un élément fonctionnel sélectionné parmi ladite lampe ultraviolette (7 ; 29), ledit diaphragme de filtration (6, 8 ; 26), et ledit filtre à charbon actif (9).

6. Dispositif selon la revendication 5, dans lequel lesdits secteurs (2^{I}, 2^{II}, 2^{III}, 2^{IV}) peuvent être accouplés ensemble en série le long d'un axe de référence (Y), en le nombre et dans l'ordre souhaités.

7. Dispositif selon l'une ou l'autre la revendication 5 ou de la revendication 6, dans lequel chaque secteur comprend des bords d'extrémité constitués par des languettes repliées (2a) dans un plan sensiblement orthogonal à l'axe de référence (Y), un élément joint d'étanchéité (4) étant placé entre les bords d'extrémité de deux secteurs contigus.

8. Diaphragme de filtration (6, 8 ; 26) pour un dispositif pour filtrer un fluide, comprenant une couche de base, qui inclut un ou plusieurs des matériaux suivants : un tissu non tissé fait de matériau synthétique, une microfibre de verre, des fibres de cellulose imprégnées, et
dans lequel, sur la couche de base du diaphragme de filtration, de l'oxynitrure de zirconium, en tant que substance photo-catalytique, et de l'argent, en tant que substance anti-pathogène, sont appliqués par l'intermédiaire de pulvérisation PVD, et
dans lequel la substance photo-catalytique constitue une matrice englobée dans laquelle sont des particules de la substance anti-pathogène.

9. Diaphragme de filtration (6, 8 ; 26) selon la revendication 8, ayant une structure multicouche qui comprend une première couche pourvue de charbons actifs ou de zéolites, et une seconde couche pourvue de la substance anti-pathogène et de la substance photo-catalytique.
